# EUROPEAN PATENT APPLICATION

(11) **EP 4 252 799 A1**
(43) Date of publication of application: **04.10.2023**
(21) Application number: 21897252.9
(22) Date of filing: 27.11.2021
(51) Int. Cl.: A61M 5/168

(54) **INTRAVENOUS INFUSION DEVICE**

(30) Priority: 27.11.2020 ES 202032583 U
(71) Applicant: Universidad De Córdoba, 14005 Córdoba (ES); Servicio Andaluz de Salud, 41071 Sevilla (ES)
(72) Inventor: REQUENA POLONIO, David, 14004 Córdoba (ES); LÓPEZ SOTO, Pablo Jesús, 14004 Córdoba (ES); RODRÍGUEZ BORREGO, María Aurora, 14004 Córdoba (ES)
(74) Representative: Pons
(86) International application number: PCT/ES2021/070855
(87) International publication number: WO 2022/112640

(57) **Abstract**

The invention discloses an intravenous infusion device (1) comprising a casing (2) having a duct (211) configured for the passage of a catheter (100) and selective restriction means for said catheter (100), where the restriction means comprise a motor (3) having a threaded shaft (31) and a locking component (4) coupled to said threaded shaft (31), said locking component (4) being disposed within a channel (212) that is provided in the casing (2) and communicates with the duct (211). Thus, when the motor (3) turns the threaded shaft (31), walls of the channel (212) in the casing (2) prevent the locking component (4) from turning, displacing the locking component (4) along the threaded shaft (31) to selectively block an opening in the duct (211) along which the catheter passes.

## Description

### OBJECT OF THE INVENTION

The object of the present invention relates to a novel device designed to measure the flow of drug administered to a patient intravenously.

### BACKGROUND OF THE INVENTION

Today, there is a wide variety of devices designed for the administration of fluid therapy to a patient. The main function of these devices is to allow a precise selection of the flow of fluid that is administered. To that end, a catheter introduced into the patient intravenously is passed through the device, which acts on the catheter to restrict the flow of fluid according to the particular needs of each case.

A large number of devices of this type which are based on different mechanisms can be found in the current state of the art. The most modern devices also have additional functions such as the selection and programming of the administered fluid therapy.

Document EP0170984 belonging to Abbott Laboratories discloses a device comprising a drip chamber and a peristaltic pump which controls the administered flow. The flow of fluid is controlled by means of a LED which emits a light beam towards a photodetector, such that it is possible to determine the passage of a droplet every time an interruption of the light beam is detected.

Document WO2014/130974 belonging to Shift Labs Inc. discloses another device for measuring the flow of a fluid applied to a patient made up of two bodies forming a clamp which is disposed around a drip chamber. The two bodies have a LED and a photodiode, respectively, which allow detecting the passage of a droplet in a manner similar to that of the preceding document.

One drawback of many of these devices is their great complexity. Therefore, there is currently a need in this field for simpler and more economical devices.

### DESCRIPTION OF THE INVENTION

The present invention solves the preceding drawbacks as a result of a novel intravenous infusion device which has a simple and compact configuration having a catheter restriction mechanism that is very simple but highly effective at the same time. Furthermore, once disposed in one position, the mechanism maintains the desired catheter restriction level without having to provide continuous power supply. This is advantageous with respect to most prior art documents, where there is a need to supply power continuously to the device in order to maintain the restriction.

The present invention discloses an intravenous infusion device fundamentally comprising a casing having a duct configured for the passage of a catheter and selective restriction means for said catheter. This device is characterized by the particular configuration of the restriction means, fundamentally comprising a stepper motor connected to a locking component.

A stepper motor is an electromechanical device which converts a series of electrical impulses into discrete angular displacements, which means that it is capable of turning a number of degrees (step or half step) depending on its control inputs. The stepper motor must be controlled by impulses from digital systems and has the advantages of presenting positioning precision and repeatability. The present invention uses a stepper motor having a threaded shaft essentially perpendicular to the duct through which the catheter passes. It should be noted that the threaded shaft does not pass through the duct directly, but rather is located in a plane parallel to the plane containing the duct.

The locking component is responsible for mechanically restricting the catheter in order to selectively limit the flow of fluid passing therethrough. To that end, the locking component is coupled by means of threading to the threaded shaft and disposed within a channel that is provided in the casing essentially parallel to the threaded shaft, with the channel communicating with the duct through which the catheter passes. As a result of this configuration, when the stepper motor turns the threaded shaft, side walls of the channel in the casing prevent the locking component from turning with the threaded shaft, such that the locking component is displaced along the threaded shaft to selectively block an opening in the duct along which the catheter passes. Accordingly, the catheter passing through the inside of said duct is restricted, thus selectively limiting the flow of fluid reaching the patient.

This configuration is particularly advantageous with respect to the systems described in the prior art documents because, once a degree of restriction of the catheter is selected, it is not necessary to supply power continuously to the restriction means. In fact, in the prior art systems which are based on the use of peristaltic pumps and similar elements, it is necessary to supply power continuously to the restriction means even when the degree of restriction of the catheter is not being modified. In contrast, in the device of the invention, once the position of the locking component corresponding to the desired degree of restriction is selected, it is possible to stop the operation of the stepper motor completely. Significant energy savings is thus achieved, allowing the use of smaller batteries in comparison with previous devices, thereby reducing the final size of the device.

In principle, the locking component can have any configuration as long as the displacement thereof along the threaded shaft causes the selective introduction or exit thereof with respect to the duct through which the catheter passes. Specifically, according to a particularly preferred embodiment of the invention, the locking component comprises a first portion having a threaded hole to receive the threaded shaft of the stepper motor and an essentially circular second portion which blocks the opening in the duct.

As for the casing, it can generally adopt any shape that is suitable for housing the different elements of the device. For example, according to a preferred embodiment of the invention, to facilitate the assembly and disassembly of the device, and to configure a compact device, the casing comprises a base and a cover coupled to one another. The coupling can be carried out in any known manner, for example by means of screws or the like.

The different elements constituting the device of the invention, particularly the duct for the passage of the catheter and the channel for the passage of the locking component, can be distributed in different manners within the casing, although they preferably constitute an integral part of one of the two parts making up same. In other words, these elements are incorporated as suitably formed portions of the casing itself. Thus, according to an even more preferred embodiment of the invention, the base comprises the duct for the passage of the catheter and the channel for the passage of the locking component.

According to another preferred embodiment of the invention, the device further comprises a flow measurement means configured to detect the flow of fluid passing through the catheter at any given time. These flow measurement means can be based, for example, on the disposition of a drip chamber where an emitter emits a beam which, after crossing a line where fluid droplets supplied to the patient fall, reaches a detector. For example, the emitter can be a LED which emits a light beam and the detector can be a photodetector. However, other different alternatives for the emission of a light beam are possible, where non-visible electromagnetic radiation, ultrasound, laser, or any other that is affected (for example, deviated) by a fluid droplet passing through, can be used. Thus, based on the signal obtained in the detector, the number of fluid droplets passing through the device can be determined. By knowing the density and surface tension of the fluid, the volume of each droplet, and therefore also the flow of fluid going through the device to the patient, can be determined.

In another preferred embodiment of the invention, the device further comprises a screen to show the flow of fluid passing through the catheter. The screen can be of any type, such as an electroluminiscent screen, a liquid crystal screen, a LED screen, or of any other type such as, for example, a screen with 7 segments. This screen will show the flow of fluid, as well as possibly other information that is relevant for the operation of the device, such as the total volume administered, programing, etc.

In principle, the screen can be located in any position of the casing, although it is preferably fixed to a parallelogram-shaped hole of the cover. In this way, the screen itself closes the normally rectangular hole, with the rear face thereof being oriented towards the inside of the device for electrical connection.

In another more preferred embodiment, the device further comprises communication means configured to communicate the flow of fluid passing through the catheter to an external device. In principle, the communication means can be of any type, although it is preferably of wireless type such as, for example, a Bluetooth or WiFi card. This allows the device to communicate with external devices such as smart telephones, tablets, and the like. A medical professional can thus know the flow of fluid that is being administered to the patient at any given time by simply consulting his/her smart telephone. To that end, the medical professional will only have to install an app designed specifically to manage communication between the device and the smart telephone.

According to another preferred embodiment of the invention, the device also comprises processing means which communicate with the flow measurement means, the screen, and the wireless communication means to manage the operation of the device. The processing means can be of any type as long as they have sufficient processing capacity to carry out the described processing. For example, the processing means can be a microcontroller, a microprocessor, an FPGA, a DSP, an ASIC, or others.

According to yet another preferred embodiment, the device of the invention further comprises a power supply battery connected to the processing means. For example, it can be of a battery that can be recharged through a conventional charger which is connected by means of a port of any suitable type.

### BRIEF DESCRIPTION OF THE FIGURES

Figure 1 shows a top perspective view of the device of the invention, in which the screen has been removed to allow visual access to the inside thereof.
Figure 2 shows a side view of the device of the invention, in which the inlet of the duct through which the catheter is disposed can be seen in detail.
Figure 3 shows a perspective view of the cover of the casing of the device of the present invention.
Figure 4 shows a perspective view of the base of the casing of the device of the present invention.
Figure 5 shows a perspective view of the locking component coupled to the stepper motor of the device of the present invention.
Figure 6 shows a diagram of the different parts making up the device of the present invention.
Figure 7 shows a view of the device of the present invention during use coupled to a catheter.

### PREFERRED EMBODIMENT OF THE INVENTION

A particular example of the device (1) of the present invention is disclosed below in reference to the figures which show the different elements making up same.

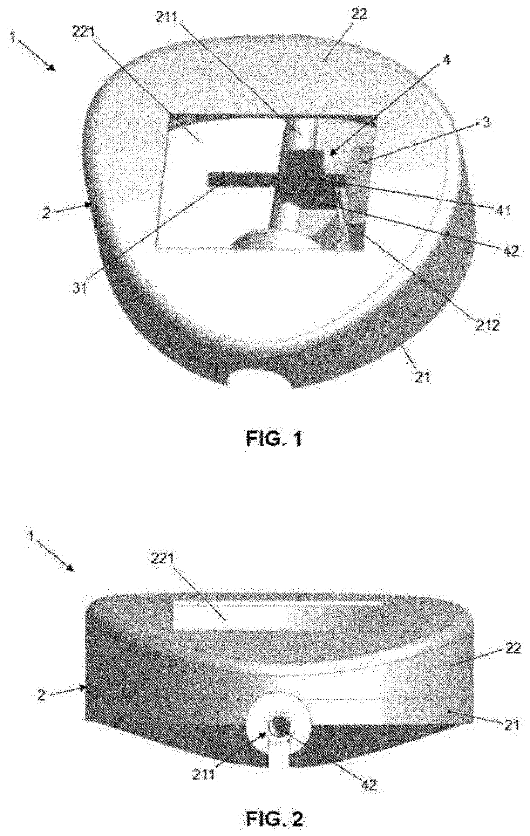

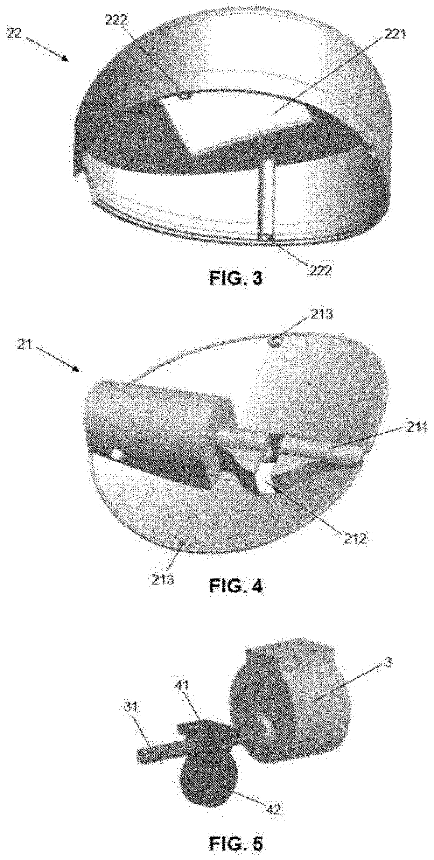

The device (1) is formed essentially by a casing (2) which houses a stepper motor coupled to a locking component (4). As can be seen in Figures 1-5, the casing (2) has an essentially cylindrical shape formed by two halves referred to as base (21) and cover (22). In this example, the base (21) comprises two holes (213) located on the periphery thereof for coupling with corresponding holes (222) of the cover (22) by means of screws or the like.

The base (21) is formed in an essentially disk-shaped with a hollow duct (211), intended for the passage of the catheter (100), going through same. The duct (211) is interrupted by a channel (212) essentially perpendicular to the duct (211), such that the catheter (100) passing through the duct (211) is exposed in the area in which it intersects the channel (212).

In turn, the cover (22) has an essentially cylindrical disk shape open at a lower end configured for coupling to the base (21). In this way, when the base (21) and the cover (22) are coupled, a space where the different components of the device are located is generated in the inside thereof. The stepper motor (3) is coupled to an upper side of the cover (22) such that a threaded shaft (31) of said motor (3) is contained in a plane parallel to the duct (211) and is perpendicular to said duct (211) at the same time. In turn, a locking component (4) is coupled to the threaded shaft (31) by means of a threaded hole disposed in a first portion (41) of said locking component (4). A second portion (42) of the locking component (4) having an essentially circular plate shape is introduced into the channel (212) of the base (21) such that the circular plate and the plane of the channel (212) are essentially coplanar. Therefore, when the stepper motor (3) is actuated to turn its threaded shaft (31), the second portion (42) housed in the channel (212) prevents the locking component (4) from turning. The locking component (4) is therefore forced to displace linearly along the channel such that it can alternate between a retracted position in which the second portion (42) is outside the opening in the duct (211) and an extended position in which the second portion (42) is inside the opening of the duct (211). Naturally, when a catheter (100) runs along the duct (211), the disposition of the locking component (4) in the extended position restricts said catheter (100), thus limiting the flow of fluid passing through the same.

The flow supplied to the patient is determined by means of flow measurement means (5) based on a drip chamber where a LED emitter emits a light beam which, after crossing a line where fluid droplets supplied to the patient fall, reaches a photodetector. Therefore, based on the signal obtained in the photodetector, the number of fluid droplets passing through the device (1), and therefore also the flow of fluid supplied to the patient, can be determined.

The cover (22) of the casing (2) furthermore has a rectangular hole (221) configured for the coupling of an LCD screen (6) or the like which allows showing the actual flow supplied to the patient.

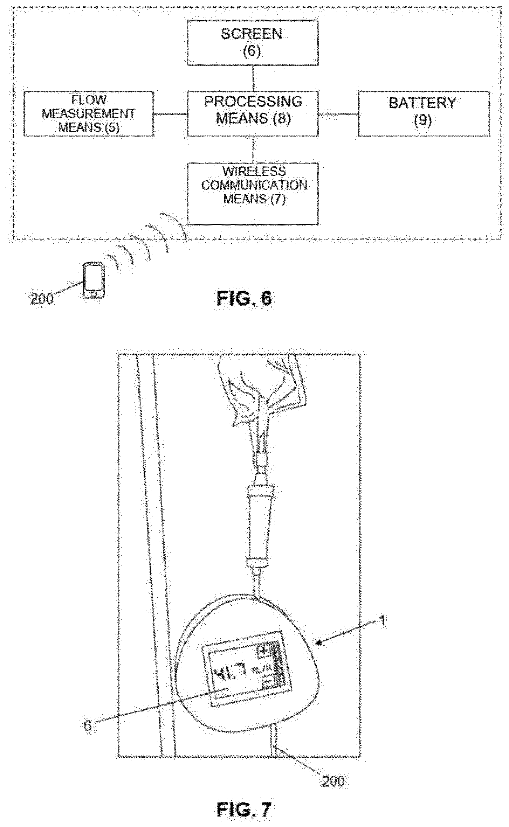

Figure 6 shows a diagram of the electric/electronic components making up the invention. Processing means (8) such as, for example, a microcontroller, are connected to the flow measurement means (5), to the screen (6), to the wireless communication means (7), for example of Bluetooth or Wifi type, and to a battery (9) that can be recharged. A medical professional can therefore control the device (1) through an app on his/her smart telephone (200) as a result of the fact that said telephone can communicate with the wireless communication means (7). The medical professional can check the flow of fluid being supplied to the patient or modify same if necessary. It is also possible to stop the supply of fluid completely in case of emergency.

Lastly, Figure 7 shows the device (1) of the invention during operation.

## Claims

1. An intravenous infusion device (1) comprising a casing (2) having a duct (211) configured for the passage of a catheter (100) and selective restriction means for said catheter (100), **characterized in that** the restriction means comprise:
- a stepper motor (3) having a threaded shaft (31) essentially perpendicular to the duct (211); and
- a locking component (4) coupled by means of threading to said threaded shaft (31), said locking component (4) being disposed within a channel (212) that is provided in the casing (2) essentially parallel to the threaded shaft (31) and communicates with the duct (211),
such that, when the stepper motor (3) turns the threaded shaft (31), walls of the channel (212) in the casing prevent the locking component (4) from turning with the threaded shaft (31), such that the locking component (4) is displaced along the threaded shaft (31) to selectively block an opening in the duct (211) along which the catheter (100) passes.

2. The intravenous infusion device (1) according to claim 1, where the locking component (4) comprises a first portion (41) having a threaded hole to receive the threaded shaft (31) of the stepper motor (3) and an essentially circular second portion (42) which blocks the opening in the duct (211).

3. The intravenous infusion device (1) according to any of the preceding claims, where the casing (2) comprises a base (21) and a cover (22) coupled to one another.

4. The intravenous infusion device (1) according to claim 3, where the base (21) comprises the duct (211) for the passage of the catheter (100) and the channel (212) for the passage of the locking component (4).

5. The intravenous infusion device (1) according to any of the preceding claims, further comprising flow measurement means (5) configured to detect the flow of fluid passing through the catheter (100) at any given time.

6. The intravenous infusion device (1) according to any of the preceding claims, further comprising a screen (6) to show the flow of fluid passing through the catheter (100).

7. The device (1) according to claim 6, where the screen (6) is fixed to a parallelogram-shaped hole (221) of the cover (22).

8. The intravenous infusion device (1) according to any of the preceding claims, further comprising communication means (7) configured to communicate the flow of fluid passing through the catheter (100) to an external device (200).

9. The intravenous infusion device (1) according to claim 8, where the communication means (7) is wireless communication means.

10. The intravenous infusion device (1) according to claim 9, where the wireless communication means (7) is a Bluetooth or WiFi card.

11. The intravenous infusion device (1) according to any of the preceding claims, further comprising processing means (8) which communicate with the flow measurement means (5), the screen (6), and the wireless communication means (7) to manage the operation of the device (1).

12. The intravenous infusion device (1) according to claim 11, where the processing means (8) is selected from: a microcontroller, a microprocessor, an FPGA, a DSP, and an ASIC.

13. The intravenous infusion device (1) according to any of the preceding claims, further comprising a power supply battery (9) connected to the processing means (8).
